(19) Europäisches Patentamt
European Patent Office
Office européen des brevets

(11) Numéro de publication: **0 580 068 A2**

(12) # DEMANDE DE BREVET EUROPEEN

(21) Numéro de dépôt: **93111274.2**

(51) Int. Cl.[5]: **C11B 9/00**, A61K 7/46

(22) Date de dépôt: **14.07.93**

(30) Priorité: **22.07.92 CH 2307/92**

(43) Date de publication de la demande:
**26.01.94 Bulletin 94/04**

(84) Etats contractants désignés:
**CH DE FR GB LI NL**

(71) Demandeur: **FIRMENICH SA**
**1, route des Jeunes**
**CH-1211 Genève 8(CH)**

(72) Inventeur: **Mimoun, Hubert, Dr.**
**Route du Stade**
**F-01630 Challex(FR)**
Inventeur: **Delay, François, Dr.**
**19, rue de la Fontenette**
**CH-1227 Carouge(CH)**
Inventeur: **Schneider, Philippe, Dr.**
**13, avenue Krieg**
**CH-1208 Geneve(CH)**

(74) Mandataire: **Salvadori, Giuseppe, Dr. et al**
**c/o Firmenich S.A.**
**Case Postale 239**
**CH-1211 Genève 8 (CH)**

(54) **Composés polycycliques nouveaux et leurs utilisations en tant qu'ingrédients parfumants.**

(57) Les composés de formule

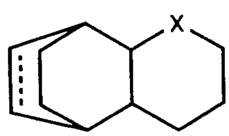

(Ia, b)

dans laquelle le symbole X sert à désigner un groupe C = O (Ia) ou $C(CH_3)$-O $C(O)CH_3$ (Ib) et la ligne pointillée indique une liaison simple ou double à la formule (Ia) et une liaison double à la formule (Ib), sont caractérisés par des notes olfactives intéressantes de type aromatique, fruité, voire ambré et peuvent de ce fait être utilisés avantageusement en tant qu'ingrédients parfumants actifs.

EP 0 580 068 A2

La présente invention a trait au domaine de la parfumerie. Plus particulièrement elle concerne l'utilisation de composés cétoniques polycycliques de formule

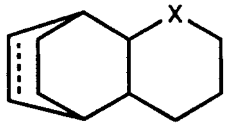

(Ia, b)

dans laquelle le symbole X sert à désigner un groupe C=O (Ia) ou $C(CH_3)$-O $C(O)CH_3$ (Ib), la ligne pointillée indiquant l'emplacement d'une liaison simple ou double dans le premier cas (Ia) et d'une liaison double dans le second cas (Ib).

Les deux composés insaturés représentés par la formule (I) sont définis comme la tricyclo[6.2.2.$0^{2,7}$]-dodéc-9-én-3-one et l'acétate de 3-méthyl-tricyclo[6.2.2.$0^{2,7}$] dodéc-9-én-3-yle. Le premier de ces composés est un produit décrit dans la littérature scientifique [voir J. Org. Chem. 1989, 54, 710-2]. Sa préparation a été en effet rapportée par Fringuelli et al. qui ont examiné toute une série de composés résultant de la réaction de Diels-Alder entre des cyclohexénones et le 1,3-cyclohexadiène. Leur étude a amené les auteurs à formuler certaines considérations théoriques quant à la diastéréosélectivité de ce type de réaction. Aucune mention particulière n'a été faite, ni aucune suggestion n'a été émise quant à l'utilité éventuelle de la cétone tricyclique obtenue. Cette même cétone a été également décrite par Hirao et al. [Chem. Comm. 1977, 577] et Nakazaki et al. [J. Org. Chem. 1980, 45, 4440-4444]. Dans ces cas, tout comme dans la référence citée plus haut, il n'est fait mention d'une quelconque utilité de la cétone en question.

L'acétate de 3-méthyl-tricyclo[6.2.2.$0^{2,7}$]dodéc-9-én-3-yle et la tricyclo[6.2.2.$0^{2,7}$]dodécan-3-one sont par contre des composés de structure nouvelle et à ce titre ils constituent également des objets de la présente invention.

Le brevet CH 616 585 (titulaire : Firmenich SA) décrit l'utilisation de tricyclo[6.2.1.$0^{2,7}$]undéc-9-én-3-one à titre d'ingrédient parfumant ou aromatisant. Ses propriétés odorantes rappellent celles développées par certaines plantes aromatiques, telles l'absinthe et le liatris.

Le brevet EP 0 007 486 (titulaire : Firmenich SA) décrit des esters de formule

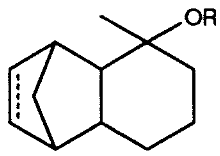

possédant une simple ou double liaison dans la position indiquée par les pointillés et dans laquelle le symbole R représente un atome d'hydrogène ou un radical acyle dérivant d'un acide carboxylique saturé ou insaturé contenant de 1 à 6 atomes de carbone. Parmi les composés mentionnés figure l'acétate de 3-méthyl-tricyclo[6.2.1.$0^{2,7}$]undéc-9-én-3-yle, lequel composé développe une odeur originale verte, caractéristique de certains des effets olfactifs de la sauge sclarée. Quoique de structure moléculaire proche, les composés de la présente invention, qui constituent formellement les homologues supérieurs des composés décrits dans les deux brevets cités, possèdent des propriétés odorantes distinctes de celles desdits composés et peuvent de ce fait trouver un emploi en tant qu'ingrédients parfumants originaux.

Nous avons découvert que, de façon surprenante, la tricyclo[6.2.2.$0^{2,7}$]dodéc-9-én-3-one sert à développer une note aromatique et fruitée avec une sous-note verte. Son caractère évoque le myrte et l'armoise avec son côté franchement thuyonique. Elle possède également un caractère floral de type géranium. Comparé à son homologue inférieur, la tricyclo[6.2.1.$0^{2,7}$]undéc-9-én-3-one, la cétone de l'invention possède un côté aromatique beaucoup moins prononcé tandis que la note florale, totalement absente dans l'undécénone, s'impose comme étant le caractère dominant. De telles caractéristiques olfactives rendent la tricyclo[6.2.2.$0^{2,7}$]dodéc-9-én-3-one de l'invention particulièrement adaptée pour la préparation de compositions masculines ainsi que pour la parfumerie fonctionnelle. L'homologue saturé de cette cétone, à savoir, la tricyclo[6.2.2.$0^{2,7}$]dodécan-3-one, possède pour sa part une odeur aromatique thuyonique très naturelle, plus élégante et thuyonique que celle de l'undécénone connue citée plus haut, accompagnée aussi d'un côté de type myrte et cyprès sans terpenes.

L'acétate de 3-méthyl-tricyclo[6.2.2.$0^{2,7}$]dodéc-9-én-3-yle présente, quant à lui, le caractère ambré propre à la note de tête de l'ambre gris, sans posséder son caractère marin. Cette note le distingue de son

homologue inférieur, lequel présente un aspect beaucoup plus chimique, presque benzoïque dans la direction de l'Ylang. Il s'est avéré en pratique que l'emploi de l'acétate de l'invention permet de compléter de façon fort harmonieuse l'effet conféré par l'Ambrox (marque enregistrée de Firmenich SA), en donnant aux compositions dans lesquelles les deux ingrédients sont incorporés une note de tête distincte qui amplifie la note de fond propre à l'Ambrox®.

Compte tenu de ce qui précède, il n'est pas étonnant de constater en pratique que les composés de l'invention ne peuvent être remplacés par les composés connus de l'art antérieur. Ce fait illustre une fois encore, si besoin était, le caractère de surprise qui entoure le plus souvent les découvertes dans le domaine de la parfumerie. Nombreux sont en effet les exemples nous montrant comment une modification apparemment mineure dans la structure moléculaire peut entraîner des modifications inattendues, souvent fondamentales dans les propriétés odorantes d'un composé donné. L'homme du métier ne dispose de ce fait d'aucun critère véritablement efficace, capable de le guider dans le choix qu'il doit opérer lors de la synthèse d'un ingrédient actif.

Les composés cétoniques polycycliques de l'invention, peuvent être employés dans des concentrations qui varient dans une gamme de valeurs assez étendue. Le parfumeur sait par expérience choisir les proportions idoines en fonction de la nature des autres coingrédients dans une composition donnée et en fonction, bien entendu, du caractère olfactif qu'il souhaite atteindre. Ainsi, des proportions de l'ordre de 5-10 à 40-50% peuvent être utilisées. Typiquement, de telles proportions peuvent être inférieures lors du parfumage de produits de consommation courants tels les savons, détergents, produits pour le soin des cheveux, cosmétiques, désodorisants corporels ou d'air ambiant ou les produits d'entretien.

Les composés de l'invention peuvent se combiner harmonieusement avec une grande variété d'autres ingrédients parfumants courants tant synthétiques que naturels. A ce titre il convient de citer par exemple, les composés décrits dans l'ouvrage de S. Arctander, Perfume and Flavor Chemicals Montclair N.J. (USA) 1969. Bien entendu, les composés de l'invention peuvent être utilisés tels quels ou, de préférence, préalablement dissous dans un solvant ou mélangés à un adjuvant courant.

Comme mentionné plus haut, l'acétate de 3-méthyl-tricyclo[6.2.2.$0^{2,7}$]dodéc-9-én-3-yle est un composé nouveau. Il peut être obtenu à partir de la tricyclo[6.2.2.$0^{2,7}$]dodéc-9-én-3-one par addition, sous les conditions d'une réaction de Grignard, d'un halomagnésien de méthyle. Le carbinol obtenu, ou 3-méthyl-tricyclo[6.2.2.$0^{2,7}$]dodéc-9-én-3-ol, peut ensuite être estérifié selon les techniques usuelles [voir à ce sujet le brevet EP 0 007 486]. Ce procédé peut être illustré par le schéma de réaction ci-après :

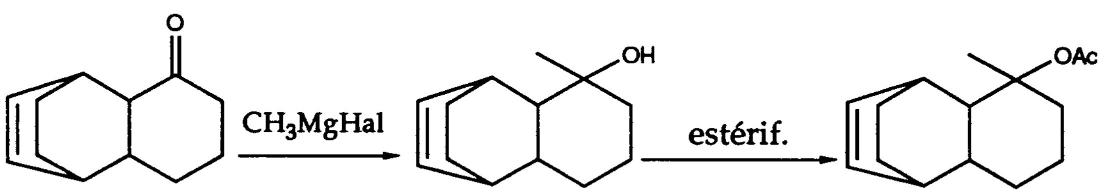


(Ia) (II) (Ib)

Ac=$CH_3C(O)$

La première étape du procédé peut s'effectuer aisément à l'aide de chlorure, bromure ou iodure de méthyl-magnésium, tandis que l'estérification a lieu par traitement du carbinol (II) avec un agent d'acétylation de formule R-Y, dans laquelle Y représente un atome d'halogène, le chlore ou le brome par exemple, ou un radical O-acétyle, et R représente un radical acétyle.

Tel que décrit dans la littérature [Fringuelli et al. op. cit.], la cétone insaturée (Ia) peut être préparée, suivant une réaction de type Diels-Alder, par addition de la cyclohex-2-én-1-one sur du cyclohexa-1,3-diène. Dans les conditions mentionnées (trichlorure d'aluminium dans des solutions de toluène à 40°C), la réaction de cycloaddition montre une diastéréosélectivité prononcée en fournissant, pour l'essentiel, le composé endo. Toutefois, lorsqu'il est fait mention dans la présente description des composés (Ia) et (Ib) en tant qu'ingredients parfumants, l'on entend se référer indifféremment aux divers isomères de configuration possible, aussi bien endo- qu'exo-.

La cétone saturée (Ia), qui comme cité précédemment est un composé nouveau, peut être préparée par hydrogénation catalytique de son homologue insaturé cité ci-dessus. Les conditions d'hydrogénation sont décrites en détail dans les exemples ci-après.

L'invention est décrite de manière plus détaillée, sans pour autant être limitée, par les exemples suivants dans lesquels les températures sont indiquées en degrés centigrades et les abréviations ont le sens usuel dans l'art.

Exemple 1

Préparation de l'acétate de 3-méthyl-tricyclo[6.2.2.0$^{2,7}$]dodéc-9-én-3-yle

a. 5,6 G (0,23 moles) de magnésium en copeaux et 16,6g de tétrahydrofurane ont été introduits sous azote dans un réacteur 4 cols équipé d'un agitateur mécanique, d'un thermomètre, d'un réfrigérant et d'une ampoule d'introduction. Une solution d'iodure de méthyle 33 g (0,232 moles) dans 100 ml de toluène a été ensuite ajoutée sous agitation à la suspension de magnésium tout en maintenant la température à environ 40° par un refroidissement extérieur. Le mélange de réaction a été maintenu à cette température pendant 20 min, puis on l'a refroidi à 0° et on a procédé à l'adjonction de 20,3 g d'endo-tricyclo[6.2.2.0$^{2,7}$]dodéc-9-én-3-one (0,115 moles) dans 100 ml de toluène. La vitesse d'addition a été réglée de façon à ce que la température du mélange de réaction ne dépasse pas 5°, puis on a laissé remonter la température jusqu'à la valeur ambiante. Après une nuit on a de nouveau refroidi à 0° et effectué l'hydrolyse par l'addition contrôlée de 100 ml d'une solution saturée de $NH_4Cl$. Après décantation, on a séparé la phase organique. Elle a été lavée deux fois avec de l'eau, séchée sur $Na_2SO_4$ et concentrée pour fournir un résidu (29 g) qui par distillation a fourni 18g (rend. 81%) de (±)-(1RS,2SR,3RS,7RS)-3-méthyl-tricyclo[6,2,2,0$^{2,7}$] dodéc-9-én-3-ol.
Eb. 110°/0,5 hPa

| | |
|---|---|
| SM : | 192($M^+$,3), 174(11), 159(2), 149(6), 131(8), 112(10), 104(10), 94(43), 80(100), 67(7), 53-(4), 43(18) |
| $^1$H-RMN : | 1,25-1,74(m, 11H) ; 1,43(s, 3H) ; 2,01(q, $^3J=9$, 1H) ; 2,05(s, 1H) ; 2,44(m, 1H) ; 2,72(m, 1H) ; 6,26 et 6,36(2t, $^3J=7,2$; 2H) δ ppm |
| $^{13}$C-RMN : | 16,0(t) ; 24,8(t) ; 25,1(t) ; 27,5(t) ; 29,9(d) ; 30,8(q) ; 34,3(t) ; 36,8(d) ; 41,0(d) ; 49,3(d) ; 72,0(s) ; 132,9(d) ; 135,4(d) δ ppm |

b. Un mélange de 6,0g (31,2 mmoles) du carbinol obtenu sous lettre a. ci-dessus, 60 ml d'acétate d'isopropényle et 0,025 g d'acide p-toluènesulfonique a été chauffé à reflux pendant 20 h. Après refroidissement, le mélange a été repris dans l'éther, lavé avec une solution aqueuse à 5% de bicarbonate de sodium, séché et concentré. Une distillation au four à boules (env. 100°/0,05 hPa) a donné 2,5 g (10,7 mmoles ; rend. 34,2%) de (±)-acétate de (1RS,2SR,3RS,7RS)-3-méthyl-tricyclo-[6.2.2.0$^{2,7}$]dodéc-9-én-3-yle.

| | |
|---|---|
| SM : | 234($M^+$,2), 219(1), 192(3), 174(33), 159(6), 146(18), 131(20), 122(17), 105(9), 94(100), 80(91), 67(8), 53(5), 43(45) |
| $^1$H-RMN : | 1,20-1,58(m, 10H) ; 1,48(s, 3H) ; 1,93(s, 3H) ; 1,97(m, 1H) ; 2,36(m, 1H) ; 2,56(dd, $^3J=8,5$ et 12, 1H) ; 2,62(m, 1H) ; 5,95 et 6,24(2t, $^3J=7,5$, 2H) δ ppm |
| $^{13}$C-RMN : | 18,2(t) ; 22,5(q) ; 23,8(t) ; 24,3(t) ; 24,7(q) ; 28,3(t) ; 30,7(t) ; 36,1(d) ; 40,7(d) ; 51,0(d) ; 82,3(s) ; 130,3(d) ; 134,3(d) ; 170,3(s) δ ppm |

Exemple 2

Préparation de la tricyclo[6.2.2.0$^{2,7}$]dodécan-3-one

Dans un ballon monocol de 100 ml on a mélangé de la tricyclo[6.2.2.0$^{2,7}$]dodéc-9-èn-3-one (10 g, 56,81 mmole) avec du méthanol (20 ml). On a ajouté ensuite du Pd/C à 5% (0,1 g) et on a effectué l'hydrogénation catalytique à pression atmosphérique pendant 1 h (agitation : 2000 tours/min). Après filtration, concentration et distillation au four à boules (P.eb. 120°/5 hPa), on a obtenu 8,7g (48,87 mmoles) de la dodécanone désirée (rend. 86%).

| | |
|---|---|
| IR(NaCl) : | 2940, 2860, 1695, 1460 $cm^{-1}$ |
| SM : | 178($M^+$,4), 160(1), 149(0,5), 110(3), 98(8), 97(100), 79(12), 67(6), 55(5), 41(9), 39(6) |
| $^1$H-RMN : | 1,25-1,88(m, 12H); 1,90-2,60(m, 6H) δ ppm |
| $^{13}$C-RMN : | 20,46(t) ; 21,40(t) ; 21,62(t) ; 25,85(d) ; 26,00(t) ; 26,96(t) ; 27,42(t) ; 29,22(d) ; 39,01(d) ; 39,87(t) ; 49,88(d) ; 215,11(s) δ ppm |

Exemple 3

On a préparé une composition de base en mélangeant les ingrédients suivants (parties en poids) :

| | |
|---|---|
| Acétate d'isobornyle | 400 |
| Acétate de terpényle | 250 |
| Bornéol crist. | 50 |
| Camphène | 200 |
| Camphre | 700 |
| Caryolan [1)] | 100 |
| Coranol [2)] | 500 |
| Eucalyptol | 50 |
| Linalol | 1000 |
| Myrcène | 50 |
| trans-2-Hexénal à 10% * | 100 |
| p-Cymène | 100 |
| Phéllandrène | 50 |
| $\alpha$-Pinène | 400 |
| Propionate d'isobornyle | 200 |
| Terpènes orange | 400 |
| Terpinène | 200 |
| Terpinéol | 100 |
| Terpinolène | 150 |
| p-Menthén-4-ol | 500 |
| Total | 5500 |

* dans le phtalate diéthylique
1) formiate de 4,4,8-triméthyltricyclo[6.3.1.0$^{2,5}$]dodéc-1-yle ; origine : Firmenich SA.
2) 4-cyclohexyl-2-méthyl-2-buténol ; origine : Firmenich SA.

Lorsqu'on ajoute à 55 parties en poids de la composition de base décrite ci-dessus 45 parties en poids de tricyclo[6.2.2.0$^{2,7}$]dodéc-9-én-3-one, on obtient une nouvelle composition dont le caractère thuyonique se situe olfactivement entre l'armoise et l'essence de cedarleaf, tout en présentant un côté naturel plaisant.
En remplaçant ladite dodécénone de l'invention par une quantité équivalente de tricyclo[6.2.1.0$^{2,7}$]undéc-9 én 3-one (composé connu de l'art antérieur ; voir brevet CH 616 585), on obtient une composition dont le caractère est beaucoup moins vert-terpénique, plus aromatique et camphré, ce qui confère à la composition une note de type absinthe et romarin.

Exemple 4

On a préparé une composition pour désodorisants corporels en mélangeant les ingrédients suivants (parties en poids) :

| | |
|---|---|
| Acétate de benzyle | 5 |
| Acétate de linalyle | 25 |
| Ald. méthylnonyl-acétique à 10%* | 10 |
| Allyl amyl glycolate | 10 |
| Ambrox® DL 1) | 10 |
| Citral | 5 |
| Ess. de citron | 35 |
| Citronellol | 5 |
| Coumarine | 35 |
| Dihydromyrcénol 2) | 90 |
| Estragol à 10%* | 35 |
| Eugénol | 5 |
| Exaltolide® 3) | 25 |
| Hédione® 4) | 35 |
| Isobutylquinoléine 10%* | 5 |
| Ess. de lavandin | 190 |
| Lilial® 5) | 45 |
| Lyral® 6) | 10 |
| Mousse cristal | 15 |
| Patchouli | 75 |
| Phénéthylol | 5 |
| Salicylate d'amyle | 40 |
| Salicylate de benzyle | 125 |
| Sandela® 7) | 75 |
| Vertofix coeur 8) | 35 |
| Ylang synth. | 15 |

| | |
|---|---|
| Zestover 9) à 10%* | 15 |
| Total | 980 |

* dans le phtalate diéthylique

1) tétraméthyl-perhydronaphtofurane ; Firmenich SA
2) International Flavors & Fragrances (IFF)
3) pentadécanolide ; Firmenich SA
4) dihydrojasmonate de méthyle ; Firmenich SA
5) 2-méthyl-3-(4-tert-butyl-1-phényl)-propanal ; Givaudan-Roure
6) 4-(4-hydroxy-4-méthylpentyl)-3-cyclohexène-1-carbaldéhyde ; IFF
7) 3-(5,5,6-triméthyl-bicyclo[2.2.1]hept-2-yl)-1-cyclohexanol ; Givaudan-Roure
8) International Flavors & Fragrances (IFF)
9) 2,4-diméthyl-3-cyclohexène-1-carbaldéhyde ; Firmenich SA

Lorsqu'on ajoute à 98 parties de la composition de base ci-dessus, 2 parties d'acétate de 3-méthyl-tricyclo-[6.2.2.0$^{2,7}$]dodéc-9-én-yle, on obtient une nouvelle composition dont la note ambrée est nettement renforcée. Il en va de même du caractère lavandin qui devient plus riche et élégant.

Exemple 5

On a préparé une composition de base pour eau-de-cologne masculine en mélangeant les ingrédients suivants (parties en poids) :

| | |
|---|---|
| Acétate de benzyle | 20 |
| Acétate de gaïol | 30 |
| Acétate de géranyle | 15 |
| Acétate de vetyvéryle | 15 |
| Anthranilol 1) | 10 |
| Bergamote | 210 |
| Cannelle Ceylan 10%* | 20 |
| Citral | 25 |
| Ess. de citron | 100 |
| Citronellol | 10 |
| Civette | 20 |
| Coumarine | 15 |

| | |
|---|---|
| Cyclosal 2) 10% * | 15 |
| Estragon 10%* | 20 |
| Eugénol | 10 |
| Exaltolide® 3) | 20 |
| Géraniol | 5 |
| Géranium Bourbon | 20 |
| Hydroxycitronellal | 15 |
| Iralia® 4) | 5 |
| Ess. de lavandin | 35 |
| Linalol | 60 |
| Ess. de mandarine | 15 |
| Mousse de chêne absolue | 20 |
| Musc C 5) | 10 |
| Neroli Portugal | 15 |
| Ess. d'origan | 5 |
| Patchouli | 25 |
| Produit AC 6) | 90 |
| Ess. de santal or. | 35 |
| Total | 910 |

* dans le phtalate diéthylique

1) méthyl N-(7-hydroxy-3,7-diméthyl-1-octényl)-anthranilate ; Takasago
2) 3-(4-isopropyl-1-phényl)-2-méthylpropanal ; Firmenich SA
3) pentadécanolide ; Firmenich SA
4) méthyl-α-ionone ; Firmenich SA
5) 4-tert-butyl-3,5-dinitro-2,6-diméthyl-1-acétophénone
6) acétate de 2,6,6,8-tétraméthyl-tricyclo[5.3.1.$0^{1,5}$]undéc-8-yle ; Givaudan-Roure

Lorsqu'on ajoute à 91 parties en poids de la composition de base ci-dessus 9 parties d'acétate de 3-méthyl-tricyclo[6.2.2.$0^{2,7}$]dodéc-9-én-3-yle on accentue remarquablement le caractère aromatique dans une direction de type sauge sclarée. Il s'est avéré que ledit ester se marie en fait parfaitement avec cette essence. Par exemple l'addition à la base indiquée d'un mélange de 6 parties d'acétate avec 3 parties de sauge sclarée permet l'obtention d'une composition dont la note de tête a plus d'impact et plus d'élégance que celle observée par l'adjonction uniquement de 9 parties de sauge sclarée.

## Revendications

1. Utilisation d'un composé cétonique polycyclique de formule

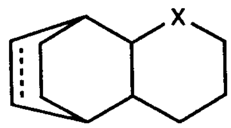

(Ia, b)

dans laquelle le symbole X sert à désigner un groupe C=O (Ia) ou $C(CH_3)$-O $C(O)CH_3$ (Ib), la ligne pointillée indiquant une liaison simple ou double pour la formule (Ia) et une liaison double pour la formule (Ib).

**2.** Utilisation selon la revendication 1, caractérisée en ce que les composés (Ia, b) se présentent sous leur forme isomérique de formule

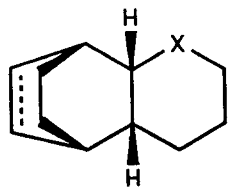

dans laquelle X sert à désigner un groupe C=O (Ia) ou $C(CH_3)$-$OC(O)CH_3$ (Ib) et la ligne pointillée est définie comme à la revendication 1.

**3.** Utilisation selon la revendication 2 de (1RS, 2SR, 7RS)-tricyclo[6.2.2.0$^{2,7}$]dodéc-9-én-3-one.

**4.** Utilisation selon la revendication 2 de l'acétate de (1RS, 2SR, 3RS, 7RS)-3-méthyl-tricyclo[6.2.2.0$^{2,7}$]-dodéc-9-én-3-yle.

**5.** Acétate de 3-méthyl-tricyclo[6.2.2.0$^{2,7}$]dodéc-9-én-3-yle.

**6.** Tricyclo[6.2.2.0$^{2,7}$]dodécan-3-one.

**7.** Composition parfumante caractérisée en ce qu'elle contient à titre d'ingrédient parfumant un composé cétonique polycyclique de formule

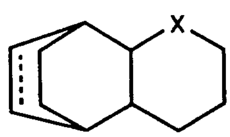

(Ia, b)

dans laquelle le symbole X sert à désigner un groupe C=O (Ia) ou $C(CH_3)$-O $C(O)CH_3$ (Ib) et la ligne pointillée est définie comme à la revendication 1.

**8.** Procédé pour conférer, renforcer ou améliorer les propriétés odorantes de parfums et produits parfumés, caractérisé en ce qu'on ajoute à une base parfumante, respectivement un produit de consommation, un composé cétonique polycyclique de formule

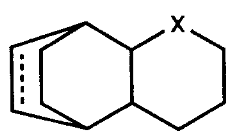

(Ia, b)

dans laquelle le symbole X sert à désigner un groupe C=O (Ia) ou $C(CH_3)$-O $C(O)CH_3$ (Ib) et la ligne pointillée est définie comme à la revendication 1.

**9.** A titre de produit parfumé selon la revendication 8, un savon, un détergent, un produit pour le soin des cheveux, un cosmétique, un désodorisant corporel ou d'air ambiant ou un produit d'entretien.